# EUROPEAN PATENT APPLICATION

(11) **EP 3 542 858 A1**
(43) Date of publication of application: **25.09.2019**
(21) Application number: 17871533.0
(22) Date of filing: 15.11.2017
(51) Int. Cl.: A61N 5/067, A61B 18/22

(54) **LIGHT RADIATING PROBE FOR PHOTODYNAMIC THERAPY EMPLOYING ENDOSCOPE**

(30) Priority: 17.11.2016 JP 2016223999
(71) Applicant: Biodynamic Research Foundation, Kumamoto-shi, Kumamoto 862-0954 (JP)
(72) Inventor: MAEDA, Hiroshi, Kumamoto-shi Kumamoto 862-0909 (JP)
(74) Representative: Prinz & Partner mbB
(86) International application number: PCT/JP2017/041131
(87) International publication number: WO 2018/092814

(57) **Abstract**

According to an aspect of the present invention, there is provided a light radiating probe which is flexible and uniformly radiates light emitted from a light scattering and radiating portion at all azimuth angles of 360° so as to enable the simultaneous radiation of light to cancers disposed at a plurality of places scattered in a wide region. The light radiating probe for photodynamic therapy according to the present invention includes an optical fiber which extends in an axial direction and through which light from a light source propagates, in which the optical fiber has a light guide portion which is formed by forming thin film cladding on a side surface of a flexible core, and a light scattering and radiating portion which is configured to scatter, with uniform intensity, light propagating through the light guide portion to a periphery of the light scattering and radiating portion in all azimuth angles with respect to an axial direction of the flexible core.

## Description

### TECHNICAL FIELD

The present invention relates to a light radiating probe for photodynamic therapy (PDT) employing an endoscope, a method of manufacturing the light radiating probe, and a photodynamic therapy apparatus including the light radiating probe for photodynamic therapy.

### BACKGROUND ART

The photodynamic therapy (PDT) is a therapy to be applied to a proliferative disease such as a cancer, by using a photosensitizing action that a photosensitive substance, that is, a photosensitizer (PS) has. The principle of the PDT has been well known for one hundred years or more after the principle was spotlighted as the subject of the Nobel Prize for Medicine in 1903. However, in spite of the fact that the principle of PDT therapy (photodynamic therapy) is extremely excellent, a clinical achievement has been considered extremely insufficient with respect to skin disease (skin tuberculosis or the like) which has been considered as a target of the PDT or a superficial cancer and the like which have become widely known over the recent twenty to thirty years.

The PDT therapy has two main drawbacks. One drawback is that a photosensitizer (PS) which has been used conventionally in the PDT therapy is a low molecular weight substance. Accordingly, the PS uniformly spreads in the entire body of a patient including an affected part and a normal part after intravenous injection, and a skin damage (photodermatosis) occurs in the normal part to which light is radiated. For example, see Patent Literature 1 which is a co-pending patent application filed by the same inventors of the present invention.

The other drawback is brought about by a situation where light having a relatively large wavelength region (for example, a HeNe laser having a peak wavelength of 633 nm) is usually used or light having a wavelength within a near infrared region is used on a trial basis to make light used in the PDT therapy easily arrive at a deep portion of a living body. That is, an optimum excitation wavelength of a photosensitizer (PS) such as Laserphyrin or Photofolin (registered trademark) is 400 to 460 nm so that the optimum excitation wavelength of the PS does not agree with the peak wavelength of the light source.

The inventors of the present invention carried out an experiment and confirmed that when a nano-particle type photosensitizer (PS) containing Zn protoporphyrin (ZnPP) is used (see Patent Document 1 above), the photosensitizer was cumulated only in a tumor part due to an enhanced permeability and retention effect (EPR effect) after a lapse of several hours from intravenous injection (IV) conducted one time (Non-patent Documents 1 to 4). The inventors of the present invention also confirmed that breast cancers and colon cancers of mice and rats were completely cured by just radiating an arbitrary light source containing a wavelength region of 400 to 460 nm one to five times to the tumor part (see Patent Document 1 and Non-patent Documents 1 and 2 above).

The conventional PDT therapy has been applied mainly to a superficial cancer (a skin cancer, a breast cancer or the like), an endothelial target cancer (bronchial lung cancer) or the like. In the latter case, an endoscopic fiberscope is introduced into an affected part (bronchial lung cancer) through an air duct, and a helium-neon (HeNe) laser beam is radiated to the affected part from the endoscopic fiberscope. However, a peak wavelength of the helium-neon laser beam is 633 nm and largely differs from an optimum excitation wavelength of a photosensitizer such as Laserphyrin or Photofolin (registered trademark). Accordingly, there is no possibility that the photosensitizer absorbs light energy and performs fluorescent light emission and hence, a singlet oxygen which kills the affected part is not also generated. Accordingly, the inventors of the present invention understand that such a therapy is not a photodynamic therapy (PDT therapy) in the true meaning of the term.

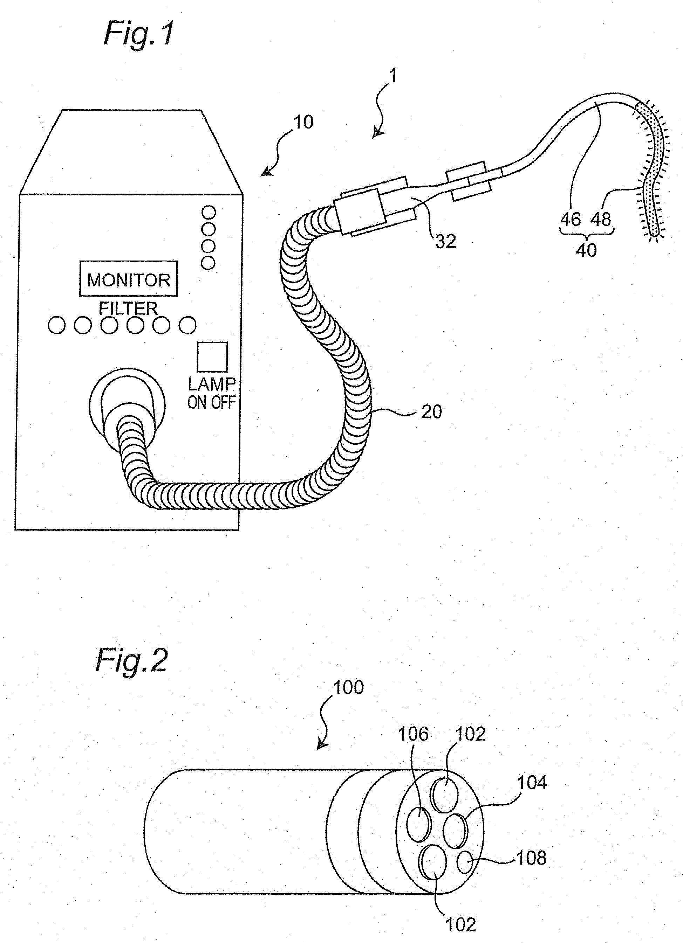

On the other hand, a generally-used endoscope is formed of three parts consisting of an operation part, an insertion part, and a connection part which connects the operation part and the insertion part to each other. As shown in Fig. 2, a distal end portion of the insertion part includes, an imaging element formed of an object lens, a CCD and the like, an optical fiber through which light from a light source apparatus propagates, an illumination lens which focuses a propagated light to an affected part, forceps openings through which treatment jigs are inserted or removed and which also function as suction openings, and a nozzle which feeds water and air. That is, the endoscope is configured such that light which passes through the illumination lens is radiated to a frontward direction (an axial direction), and the imaging element observes an affected part disposed in the frontward direction in the same manner through the object lens. In the case where an affected part does not exist in the frontward direction, the endoscope is operated such that the insertion portion per se is bent so that the distal end portion is disposed in the frontward direction of the affected part. In both cases, light from the endoscope is radiated in the frontward direction from the distal end portion of the insertion part. Further, the generally-used endoscope is designed to observe an affected part disposed in the frontward direction of the distal end portion of the insertion part, and is not designed to make use of PDT therapy.

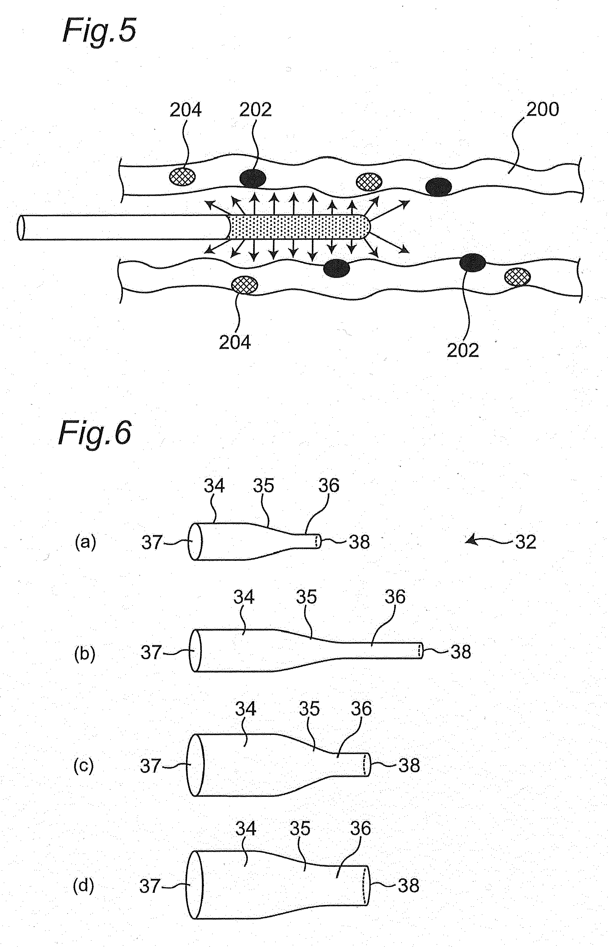

In Patent Document 2, a laser probe which is used in PDT therapy is described. However, an optical fiber to be used in working is a plastic cladded quartz core optical fiber or an all quartz optical fiber where both a core and a cladding are made of quartz (see paragraph[0030] and Fig. 5). Accordingly, a therapy target part is limited to, for example, a tubular organ such as a nasal cavity, a throat part or an uterine cervix. That is, the optical fiber having the quartz core is hard and is easily broken and hence, it is extremely difficult and, further, dangerous to insert the laser probe into a deep portion of a hollow organ such as the colon. Accordingly, it is strongly requested to provide a flexible light radiating probe having flexibility which can radiate light to a cancer affected part which exists in a deep part of a hollow organ.

On the other hand, as shown in Fig. 5, with respect to many cancers such as a colon cancer or a bladder cancer, for example, a cancer does not exist only at one place but exists in a wide area in a scattered manner simultaneously along a hollow organ. Accordingly, it is desirable to provide a light radiating probe which uniformly radiate light at all azimuth angles of 360 degrees from a side surface within a substantial length range so as to enable the simultaneous radiation of light to cancers which spread at a plurality of places in a wide region. However, the laser probe according to Patent Document 2 merely protrudes frontward from a hand piece by a slight distance (see Fig. 5 of Patent Document 2). Accordingly, the laser probe according to Patent Document 2 cannot simultaneously radiate light to cancers at a plurality of places scattering in a wide region along a hollow organ.

The inventors of the present invention have submitted a large number of papers besides Patent Document 1 and Non-patent Documents 1 to 4, which are previously mentioned (Non-patent Documents 5 to 11).

### PRIOR ART DOCUMENTS

### PATENT DOCUMENTS

Patent Document 1: WO 2013/035750
Patent Document 2: JP 2005-087531 A

### NON-PATENT DOCUMENTS

Non-patent Document 1: Journal of Japanese Society for Molecular Imaging No.9, 3-10 (2015), "Large expectation on innovative PDT by a nano probe having an EPR effect" (Hiroshi Maeda, J Fang, Hideaki Nakamura)
Non-patent Document 2: Future Science OA (2015), "Photodynamic therapy based on tumor-targeted polymer-conjugated zinc protoporphyrin and irradiation with xenon light", (J. Fang, L. Liao, H. Yin, H. Nakamura, V. Subr, K. Ulbrich, H. Maeda) http://www.future-science.com/doi/pdf/10.4155/fso.15.2, published online (2015)
Non-patent Document 3: Cancer Science 104, 779-789 (2013), "Tumor vasculature, free radicals, and drug delivery to tumors via the EPR effect", (H. Maeda)
Non-patent Document 4: Microcirculation 23,173-182 (2016), "A retrospective 30 years after discovery of the EPR effect of solid tumors: treatment, imaging, and next-generation PDT - problems, solutions, prospects", (H. Maeda. K. Tsukigawa, J. Fang)
Non-patent Document 5: Cancer Science 100, 2426-2430 (2009), "Enhanced delivery of macromolecular antitumor drugs to tumors by nitroglycerin application", (T. Seki J. Fang, H. Maeda)
Non-patent Document 6: Advanced Drug Delivery Review, 65, 71-79 (2013), "The EPR effect for macromolecular drug delivery to solid tumors: improved tumor uptake, less systemic toxicity, and improved tumor imaging in vivo", (H. Maeda, H. Nakamura, J. Fang)
Non-patent Document 7: Journal Controlled Release 165, 191-198 (2013), "Micelles of zinc protoporphyrin conjugated to N-(2-hydroxypropyl)methacrylamide (HPMA) copolymer for imaging and light-induced antitumor effects in vivo", (H. Nakamura, L. Liao, Y. Hitaka, K. Tsukigawa, V. Subr, J. Fang, K. Ulbrich, H. Maeda)
Non-patent Document 8: Therapeutic Delivery (Future Science) 5 (6), 627-630 (2014), "Emergence of EPR effect theory and development of clinical applications for cancer therapy", (H. Maeda)
Non-patent Document 9: European Journal Pharmaceutical Biopharmaceutics, 81, 540-547 (2012), "HSP32 (HO-1) inhibitor, copoly (styrene-maleic acid)-zinc protoporphyrin IX, a water-soluble micelle as anticancer agent: In vitro and in vivo anticancer effect", (J. Fang, K. Greish, H. Qin, H. Nakamura, M. Takeya, and H. Maeda)
Non-patent Document 10: Expert Opinion on Drug Delivery 12 (1), 53-64 (2015), "Development of next-generation macromolecular drugs based on the EPR effect: challenges and pitfalls", (H. Nakamura, J. Fang and H. Maeda)
Non-patent Document 11: European Journal Pharmaceutical Biopharmaceutics, 89, 259-270 (2015), "Effect of different chemical bonds in pegylation of zinc protoporphyrin that affects drug release, intracellular uptake, and therapeutic effect in the tumor", (K. Tsukigawa, H. Nakamura, J. Fang, M. Otagiri, H. Maeda)

### SUMMARY OF THE INVENTION

### PROBLEMS TO BE SOLVED BY THE INVENTION

The present invention has been made in view of the above-mentioned drawbacks, and according to an aspect of the present invention, there is provided a light radiating probe which is flexible and uniformly radiates light from a side surface of a substantial length range (for example, 20 cm to 30 cm) at all azimuth angles of 360 degrees so as to enable the simultaneous radiation of light to cancers disposed at a plurality of places scattered in a wide region.

### MEANS FOR SOLVING THE PROBLEMS

According to an aspect of the present invention, there is provided a light radiating probe for photodynamic therapy. The light radiating probe for photodynamic therapy includes an optical fiber which extends in an axial direction and through which light from a light source propagates, in which the optical fiber has a light guide portion which is formed by forming thin film cladding on a side surface of a flexible core, and a light scattering and radiating portion which is configured to scatter, with uniform intensity, light propagating through the light guide portion to a periphery of the light scattering and radiating portion in all azimuth angles with respect to an axial direction of the flexible core.

According to one embodiment of the present invention, the light scattering and radiating portion has a length which corresponds to a length of 1 cm or more of an affected part therapy target portion in an axial direction and is configured to radiate the light propagating from the light guide portion to an entire area near the affected part therapy target portion in all azimuth angles of 360 degrees, and a peak wavelength of the light from the light source is included in an optimum excitation wavelength region of a desired photosensitizer used in photodynamic therapy.

According to another embodiment of the present invention, the light scattering and radiating portion further includes a spirally wound rod.

Preferably, the rod is a rod-shaped endoscopic fiberscope.

According to still another embodiment of the present invention, the light radiating probe for photodynamic therapy further includes a covering part which covers the light scattering and radiating portion and the rod.

According to another aspect of the present invention, there is provided a photodynamic therapy apparatus including a light source which radiates light. The photodynamic therapy apparatus includes: a first optical fiber through which light from the light source propagates and including an emitting end surface which has a first cross-sectional area; an optical condenser adapter having a condenser incident end surface through which light from the first optical fiber propagates and which is substantially adapted to the emitting end surface of the first optical fiber, and a condenser emitting end surface which is smaller than the emitting end surface of the first optical fiber and is substantially adapted to an incident end surface of a second optical fiber; and the second optical fiber through which the light from the optical condenser adapter propagates and including an incident end surface substantially adapted to a second cross-sectional area of the emitting end surface of the optical condenser adapter, in which the second optical fiber has: a light guide portion which is formed by forming a thin film cladding on a side surface of a flexible core; and a light scattering and radiating portion configured to scatter, with uniform intensity, light which propagates through the light guide portion to a periphery of the light scattering and radiating portion in all azimuth angles with respect to an axial direction of a flexible core.

According to one embodiment of the present invention, the light scattering and radiating portion has a length which corresponds to a length of 1 cm or more of an affected part therapy target portion in an axial direction and is configured to radiate the light propagating from the light guide portion to an entire area near the affected part therapy target portion in all azimuth angles of 360 degrees, and a peak wavelength of the light from the light source is included in an optimum excitation wavelength region of a desired photosensitizer used in photodynamic therapy.

According to another embodiment of the present invention, the first and second optical fibers are formed of a plastic optical fiber having flexibility, and the optical condenser adapter has: a glass core whose cross-sectional area is continuously decreased between an incident end surface having a first cross-sectional area and an emitting end surface having a second cross-sectional area; and a thin film cladding which is formed on a side surface of the glass core.

Preferably, the light scattering and radiating portion of the second optical fiber further includes a spirally wound rod.

According to still another embodiment of the present invention, the rod is a rod-shaped endoscopic fiberscope.

According to yet another embodiment of the present invention, the photodynamic therapy apparatus further includes a covering part which covers the light scattering and radiating portion and the rod.

According to still another aspect of the present invention, there is provided a method of manufacturing a light radiating probe for photodynamic therapy. The method includes the steps of: providing an optical fiber which is formed by forming a thin film cladding on a side surface of a flexible core; forming a light scattering and radiating portion by processing a side surface of a distal end portion of the optical fiber so as to scatter, with uniform intensity, light which propagates to the optical fiber at a distal end portion of the optical fiber in all azimuth angles; and winding the light scattering and radiating portion around a rod.

According to one embodiment of the present invention, the light scattering and radiating portion has a length which corresponds to a length of 1 cm or more of an affected part therapy target portion in an axial direction and is configured to radiate the light propagating from the light guide portion to an entire area near the affected part therapy target portion in all azimuth angles of 360 degrees, and a peak wavelength of the light from the light source is included in an optimum excitation wavelength region of a desired photosensitizer used in photodynamic therapy.

According to another embodiment of the present invention, the step of forming the light scattering and radiating portion by processing the side surface of the distal end portion of the optical fiber includes any one of the steps of: exposing the flexible core by removing the thin film cladding disposed on the side surface of the distal end portion of the optical fiber and roughening a surface of the flexible core; making the side surface of the thin film cladding disposed on the side surface of the distal end portion of the optical fiber opaque using a solvent,; and adhering fine powder on the side surface of the flexible core exposed by removing the thin film cladding disposed on the side surface of the distal end portion of the optical fiber.

According to still another embodiment of the present invention, the method further includes a step of covering the light scattering and radiating portion and the rod with a resin material.

### EFFECTS OF THE INVENTION

According to the aspect of the present invention, it is possible to provide a flexible light radiating probe which can uniformly radiate light from the light scattering and radiating portion having a length of 1 cm or more in an axial direction in all azimuth angles of 360 degrees so as to enable simultaneous radiation of light to cancers at a plurality of places which spread in a wide region.

### BRIEF DESCRIPTION OF THE DRAWINGS

Fig. 1 is a schematic view of a photodynamic therapy apparatus including a light radiating probe for photodynamic therapy according to one embodiment of the present invention.
Fig. 2 is a schematic view showing a distal end portion of a light radiating probe according to the prior art.
Fig. 3 is a cross-sectional view of the light radiating probe for photodynamic therapy according to one embodiment of the present invention.
Fig. 4 is a schematic view showing a light scattering and radiating portion of the light radiating probe for photodynamic therapy shown in Fig. 3.
Fig. 5 is a conceptual view of a state where the light radiating probe for photodynamic therapy shown in Fig. 3 is inserted into a colon and the probe radiates light to a superficial cancer tissue and a lower layer cancer tissue in the colon.
Fig. 6 is a schematic view of an optical condenser adapter for condensing light by connecting first and second optical fibers to the optical condenser adapter.
Fig. 7 (a) is a schematic view showing a light radiating probe for photodynamic therapy according to a modification in a state where a light scattering and radiating portion is wound around a rod, Fig. 7(b) is a schematic view of the light radiating probe for photodynamic therapy in a state where the light radiating probe and the light scattering and radiating portion shown in Fig. 7(a) are covered by a thermosetting plastic sheath, and Fig. 7(c) is a schematic view showing a state where the thermosetting plastic sheath shown in Fig. 7(b) is shrunken by heating.
Fig. 8 is a conceptual view substantially equal to Fig. 5 when the light radiating probe for photodynamic therapy according to the modification is inserted into the colon.
Fig. 9 is a schematic view showing the light radiating probe in an ON state (an upper portion of the drawing), a state where the light scattering and radiating portion in an OFF state is inserted into the colon through the anus of a mouse (an intermediate portion of the drawing) and a state where the light scattering and radiating portion inserted into the colon through the anus of the mouse is brought into an ON state (a lower portion of the drawing).

### EMBODIMENTS OF THE INVENTION

With reference to attached drawings, a photodynamic therapy apparatus including a light radiating probe for photodynamic therapy according to one embodiment of the present invention is described in detail hereinafter. The photodynamic therapy apparatus 1 according to the present invention roughly includes: as shown in Fig. 1, a light source apparatus 10, a flexible fiber optics (first optical fiber) 20, a joint jig 30, and a light radiating probe for photodynamic therapy (second optical fiber) 40. The light radiating probe for photodynamic therapy 40 is used for curing an affected part such as cancer cells of mainly hollow organs (esophagus, enteron, stomach, bladder or uterus or the like), but not limited thereto.

The photodynamic therapy apparatus 1 of the present invention is configured such that light emitted from the light source apparatus 10 propagates through the fiber optics 20, and propagates to the light radiating probe for photodynamic therapy 40 through the joint jig 30. Although it is optional, as described later in detail, the joint jig 30 may have an optical condenser adapter 32 which increases photo intensity per unit area between the fiber optics 20 and the light radiating probe for photodynamic therapy 40.

The light source apparatus 10 may be a light source apparatus which has a xenon arc lamp, a tungsten lamp, or a multicolor LED light source. It is preferable to use a light source apparatus which emits light in a wide wavelength region ranging from a near ultraviolet ray to a near infrared ray. It is more preferable to use a light source apparatus where a peak wavelength is included in an optimum excitation wavelength region of a photosensitive substance, that is, a photosensitizer (PS) used in a photodynamic therapy (PDT). Photodynamic therapy (PDT) is a therapy where singlet oxygen is generated by radiating light having an optimum excitation wavelength region to a photosensitive substance, that is, a photosensitizer (PS) by making use of a photosensitizing action which the photosensitizer has, and an affected part such as cancer cells or the like is cured (killed) by singlet oxygen. In this manner, with the use of the light source which generates light of a wide wavelength region including 400nm to 460nm which is an optimum excitation wavelength region of a desired photosensitizer (PS) used in photodynamic therapy (for example, Laserphyrin and Photofolin), singlet oxygen is efficiently generated in the photosensitizer so that an affected part such as cancer cells can be effectively cured. Although the light source apparatus 10 is not limited to such a light source apparatus, for example, a light source apparatus (EVIS CLV-U20D, registered trademark) made by OLYMPUS Corporation may be used.

Optionally, the light source apparatus 10 may be used in such a manner that an excitation light having Gauss distribution intensity within a range of 400 nm to 460 nm is emitted by combining a blue LED or an ultraviolet LED with a fluorescent substance. In this manner, by selecting the LED light source apparatus 10 in conformity with a desired photosensitizer used in a photodynamic therapy, a more compact, light-weighted and inexpensive photodynamic therapy apparatus 1 can be realized, and a curing effect of a photosensitizer can be optimized.

A conventional light radiating probe 100 is described with reference to Fig. 2. Fig. 2 is a perspective view showing a distal end portion of a light radiating probe 100. The light radiating probe 100 basically has an illumination lens 102 which radiates light to an affected part, an object lens (including a CCD element) 104, forceps openings 106 which are used for inserting and removing treatment jigs and functioning as suction openings, and a nozzle 108 which feeds water or air. That is, the conventional optical light radiating probe 100 is formed of the illumination lens 102 which radiates light to an affected part. Accordingly, light can be radiated only in a longitudinal direction (a frontward direction) of the light radiating probe 100 and hence, light cannot be radiated to an entire area near an affected part therapy target portion such as a cancer affected part at all azimuth angles of 360 degrees whereby the conventional light radiating probe 100 is not suitable for being used in the photodynamic therapy.

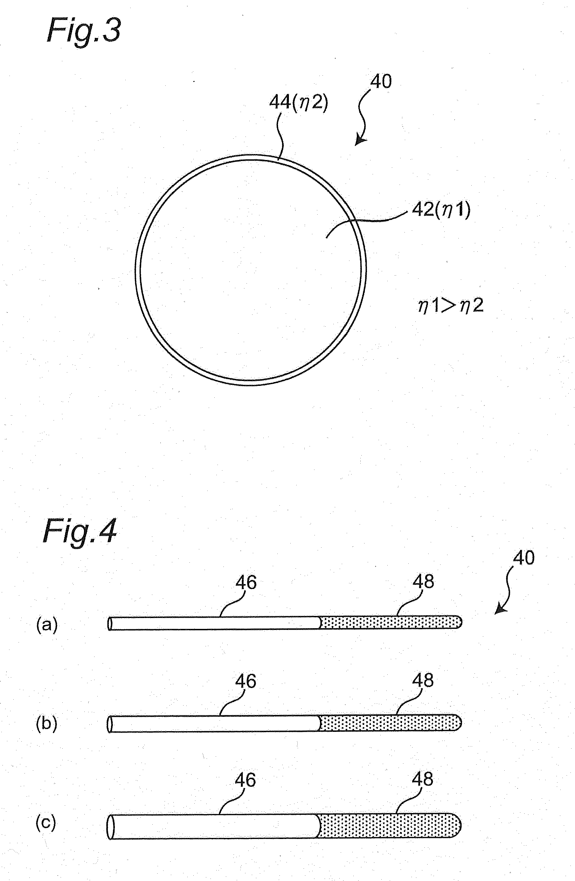

Next, the light radiating probe 40 according to the present invention (hereinafter simply referred to as "light radiating probe") is described with reference to Fig. 3 and Fig. 4. In general, it is preferable to form the light radiating probe 40 using an arbitrary constitutional material having flexibility. As shown in Fig. 3 which is a cross-sectional view, for example, the light radiating probe 40 may be an optical fiber cable which is formed by covering a core member 42 made of an acrylic resin or the like by a cladding 44 made of a transparent fluoro-resin layer or the like (thin film cladding). A refractive index (η2) of the cladding 44 is designed to be smaller than a refractive index (η1) of the core member 42 (η1 > η2). Accordingly, light which propagates to the core member 42 is confined in the core member 42 due to the total reflection of the light on a boundary surface between the core member 42 and the cladding 44 and hence, propagates toward a distal end portion in an axial direction while repeating the total reflection on the boundary surface between the core member 42 and the cladding 44. However, it is not always necessary for the light radiating probe 40 to have flexibility depending on a usage, and the core member 42 may be formed using glass or the like. Further, the light radiating probe 40 according to the present invention may adopt a more inexpensive step index multimode optical fiber, but not limited thereto.

As shown in Fig. 4, the light radiating probe 40 according to the present invention includes: a light guide portion 46 which receives light from the optical condenser adapter 32 and where side surface of the core member 42 is covered by a cladding 44 at a distal end portion of the light guide portion 46; and a light scattering and radiating portion 48 which scatters, with uniform intensity, light which propagates through the light guide portion 46 to a periphery of the light scattering and radiating portion 48 over all azimuth angles with respect to an axial direction of the light radiating probe 40. The light scattering and radiating portion 48 has a length of 1 cm or more (may be also 20 cm to 30 cm) in a longitudinal direction from the distal end portion of the light radiating probe 40. The light scattering and radiating portion 48 preferably has a length which corresponds to a length of an affected part therapy target portion in an axial direction. The light scattering and radiating portion 48 is configured to radiate light which propagates from the light guide portion 46 to an entire area near the affected part therapy target portion such as a cancer affected part in all azimuth angles of 360 degrees.

To be more specific, the light radiating probe 40 according to the present invention includes the light guide portion 46 and the light scattering and radiating portion 48. It is sufficient that a diameter of the light radiating probe 40 be 0.1 mm or more. However, the diameter of the light radiating probe 40 is not limited to such a value. As shown in Fig. 4 (a) to Fig. 4 (c), the diameter of the light radiating probe 40 may be 2 mm, 3 mm or 5 mm.

The light scattering and radiating portion 48 of the light radiating probe 40 can be manufactured using various techniques. For example, the light scattering and radiating portion 48 may be manufactured by forming scratches by grinding or rubbing the cladding 44 disposed on the distal end portion of the light radiating probe 40 in a random direction using, for example, a sand paper (coarseness of grit being, for example, a coarse grit of #100, a middle grit of #200 or a fine grit of #400) or a rasp or the like.

Additionally or selectively, the core member 42 is immersed in a soluble solvent (for example, acetone or chloroform or the like) in which a resin which forms the cladding 44 disposed on the distal end portion of the light radiating probe 40 is dissolved and, thereafter, the cladding 44 is immersed in a non-soluble solvent for a short time, and is dried naturally so that a surface of the cladding is made opaque thus forming the light scattering and radiating portion 48. In such an operation, a resin which forms the cladding 44 of the light scattering and radiating portion 48 is partially removed. Accordingly, due to a change in physical characteristics including the reduction of a refractive index, a light confining effect is decreased and hence, it is possible to scatter, with uniform intensity, light from the entire light scattering and radiating portion 48 to a periphery of the light scattering and radiating portion 48 in all azimuth angles.

Additionally or alternatively, the cladding 44 which is formed on the distal end portion of the light radiating probe 40 is wholly or partially removed and, thereafter, particles (including fine particles) of alumina, copper, silver, iron, an alloy of these metals or other arbitrary metal; ceramic; titanium dioxide; celite; white soil powder; or the like may be suspended or dispersed at an appropriate concentration in an acrylic resin or the like which forms a side surface of the core member 42. By applying such a treatment, light which propagates from the core member 42 of the light scattering and radiating portion 48 is subjected to diffused reflection by the above-mentioned particles (including fine particles) so that it is possible to scatter light from the entire light scattering and radiating portion 48 to a periphery in all azimuth angles with uniform intensity by diffused reflection on the above-mentioned particles (including fine particles).

Fig. 5 is a schematic view of a state where, for example, the light radiating probe 40 according to the present invention shown in Fig. 4(c) is inserted into the colon 200, a photosensitizer (PS) is injected, and light including an optimum excitation wavelength region is radiated to a superficial cancer tissue 202 and a lower layer cancer tissue 204 in the colon 200. In general, the lower layer cancer tissue 204 is a tumor nodule which is difficult to recognize with naked eye. However, the inventors of the present invention have confirmed that not only the lower layer cancer tissue 204 of the colon 200 but also lower layer cancer tissues 204 of an esophageal, a stomach, an intestinal tract, a bladder cavity, a peritoneum, an uterus, an abdominal cavity and other body cavities can be detected by phosphorous detection due to an EPR effect of the photosensitizer. In this manner, according to the light radiating probe 40 of the present invention, it is possible to properly capture a lower layer cancer tissue of a body cavity which cannot be easily recognized with the naked eye, and the lower layer cancer tissue 204 can be killed more efficiently.

According to the embodiment of the present invention, as described previously, the joint jig 30 may have the optical condenser adapter 32 between the fiber optics 20 and the light radiating probe for photodynamic therapy 40. The optical condenser adapter 32 is provided for increasing light intensity per unit area. The optical condenser adapter 32 may be formed of, for example, a glass core formed using a hard material such as glass, and a clad thin film having a smaller refractive index than the glass core. Further, as shown in Figs. 6(a) to 6(d), the optical condenser adapter 32 includes a cylindrical large-diameter portion 34, a small-diameter portion 36, and a neck portion 35 disposed between the cylindrical large-diameter portion 34 and the small-diameter portion 36, and has an incident end surface 37 disposed on a left side in the drawing, and an emitting end surface 38 disposed on a right side in the drawing. The incident end surface 37 of the large-diameter portion 34 of the optical condenser adapter 32 has the same size and shape as an emitting end surface (not shown in the drawing) of the fiber optics (first optical fiber) 20 and is configured to be joined (coupled) to the emitting end surface such that the incident end surface 37 is substantially adapted to (coupled to) the light radiating surface. On the other hand, the emitting end surface 38 of the small-diameter portion 36 of the optical condenser adapter 32 has the same size and shape as an incident end surface (not shown in the drawing) of the light radiating probe (second optical fiber) 40 and is configured to be joined (coupled) to the incident end surface such that the emitting end surface 38 is substantially adapted to the incident end surface.

The optical condenser adapter 32 can be easily manufactured by melting a portion of Pyrex glass using a burner, and by pulling the portion in directions in which the large-diameter portion 34 and the small-diameter portion 36 are separated from each other, the portion having a diameter of 10 mm, for example, and corresponding to the neck portion 35. The optical condenser adapter 32 according to the present invention can be manufactured by softening by heating a polymer resin having high transparency such as Lucite (registered trademark), polypropylene, polyethylene, polyvinyl alcohol or polystyrene besides glass.

Accordingly, the optical condenser adapter 32 is formed such that light intensity per unit area of light which propagates to the incident end surface 37 of the large-diameter portion 34 is increased along with the reduction of a cross-sectional area in a path ranging from the large-diameter portion 34 to the small-diameter portion 36 by way of the neck portion 35. With such a configuration, it is possible to radiate stronger light from the light scattering and radiating portion 48 to an entire area near an affected part therapy target portion such as a cancer affected part in all azimuth angles of 360 degrees. In Fig. 6, the optical condenser adapters 32 having various end surface diameters and various axial lengths are illustrated. However, provided that the optical condenser adapter 32 is configured such that light intensity per unit area is increased, the optical condenser adapter 32 having an arbitrary end surface diameter and an arbitrary axial direction length may be used.

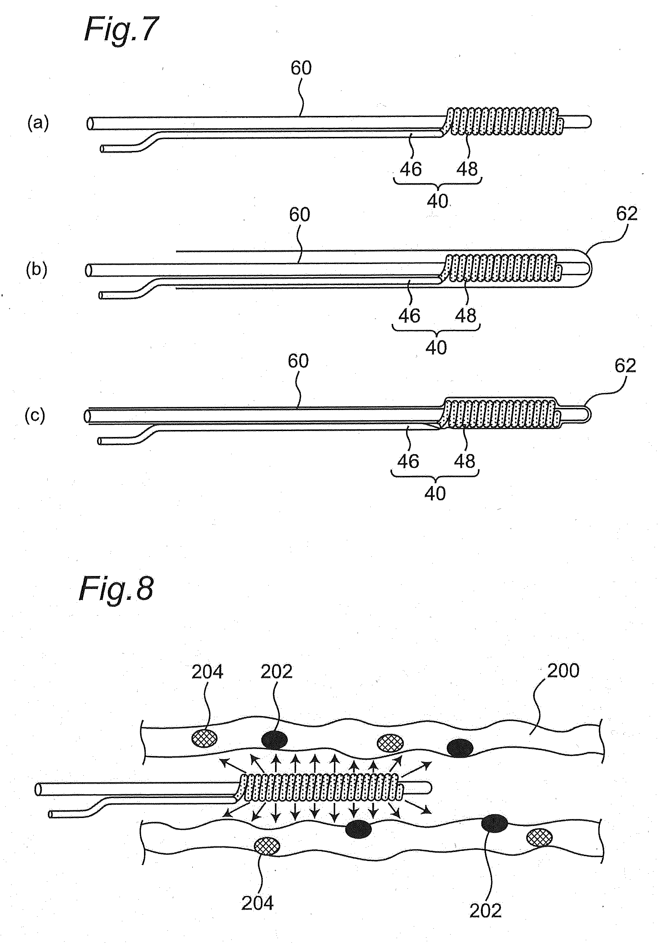

Modifications of the above-mentioned embodiment are described with reference to Fig. 7 and Fig. 8. The light radiating probes 40 shown in Fig. 7 are formed such that the light scattering and radiating portion 48 of the light radiating probe 40 according to the above-mentioned embodiment is wound around a rod (or a simple bar-like member) 60 in a spiral shape or in a coil shape. It is preferable that the rod 60 be made of a material harder than a material for forming the light radiating probe 40 having flexibility. For example, the rod 60 may be formed of endoscope fiber optics. Fig. 8 is a schematic view showing a state where the light radiating probe 40 according to the modification shown in Fig. 7(a) is inserted into the colon, a photosensitizer (PS) is injected, and light including an optimum excitation wavelength region is radiated to the superficial cancer tissue 202 and the lower layer cancer tissue 204 of the colon.

As shown in Fig. 7(a), the light scattering and radiating portion 48 is wound around the rod 60 in a coil shape. Accordingly, light which propagates from the light guide portion 46 can be radiated to an entire area near an affected part therapy target portion such as a cancer affected part in all azimuth angles of 360 degrees along a desired length in a longitudinal direction. Although it also depends on winding density, compared to the light scattering and radiating portion 48 according to the above-mentioned embodiment shown in Fig. 5, the light scattering and radiating portion 48 according to the modification shown in Fig. 7(a) can radiate stronger (more concentrated) light to an affected part therapy target portion and hence, the light scattering and radiating portion 48 according to the modification shown in Fig. 7(a) can expect a higher therapeutic effect. Further, the light radiating probe 40 is formed by winding the light scattering and radiating portion 48 around the rod 60 according to the previously-mentioned embodiment in a coil shape. Accordingly, a length of the light scattering and radiating portion 48 can be easily adjusted corresponding to a length of the affected part therapy target portion in the longitudinal direction. Accordingly, it is possible to extremely easily manufacture the light scattering and radiating portion 48 suitable for a length of an affected part therapy target portion to be radiated by light.

As shown Fig. 7(b), the light radiating probe 40 is formed such that a light scattering and radiating portion 48 is wound around a rod 60 in a coil shape and, then, the rod 60 and the light scattering and radiating portion 48 are covered by a sheath (cover film) 62 made of a thermosetting resin (for example, a polyvinyl-based resin), and the sheath 62 is thermally shrunken by applying heat to the sheath 62 by a hot air generating device (for example, a dryer or the like). As shown Fig. 7(c), a light radiating probe 40 may be formed such that a rod 60 and a light scattering and radiating portion 48 are integrally fixed to each other by a protective film (sheath) 62. In such a configuration, it is preferable that a coating adhesive agent be applied to outer surfaces of the rod 60 and to the light scattering and radiating portion 48 or to an inner surface of the sheath 62 in advance so as to acquire the adhesion between the rod 60, the light scattering and radiating portion 48 and the sheath 62 with certainty. With such a configuration, when the light scattering and radiating portion 48 shown in Fig. 7(c) is inserted into a body cavity such as the colon, it is possible to prevent the removal of the light scattering and radiating portion 48 from the rod 60 and hence, it is possible to make the light scattering and radiating portion 48 reach an affected part therapy target portion which forms a target with certainty.

A protective film 62 substantially equal to the protective film shown in Fig. 7(c) can be easily manufactured by winding the light scattering and radiating portion 48 around the rod 60 in a coil shape and, thereafter, by immersing the rod 60 and the light scattering and radiating portion 48 into a melted solution of a polyvinyl alcohol-based resin or an acrylic resin or into a tacky solution made of a synthetic resin and, then, by drying.

As has been described heretofore, the present invention has the following advantageous effects.

It is important for a general-use optical fiber to transmit light frontward without loss. On the other hand, in the present invention, light from the distal end portion of the light radiating probe 40 is radiated to a peripheral portion of a hollow organ part (for example, an oral cavity, an esophageal, a stomach, an intestinal tract, an abdominal cavity, a bladder cavity, a peritoneum, a diaphragm, an uterus, a thoracic cavity, a bronchial tube, upper and lower air ducts, a pharynx, a liver surface and the like) of 360°. By performing fluorescent light emission by exciting photosensitizer (PS) molecules of a nano-size selectively accumulated in a local cancer tissue by an EPR effect, the position of the lower layer cancer tissue 204 which cannot be easily recognized with naked eye can be easily specified and, at the same time, singlet oxygen which is one of active oxygens is generated from the photosensitizer (PS) molecules so that the light radiating probe 40 can exhibit an anti-cancer effect.

Accordingly, in the photodynamic therapy (PDT), it is necessary to radiate light in all azimuth angles of 360° toward a tube wall (an intestinal tract, an abdominal wall, a chest wall) which forms a peripheral portion behind an affected part rather than advancing the light straight in an area near the affected part. Accordingly, it is preferable that the light radiating probe 40 according to the present invention be formed using a wire-like (string-like) optical fiber having high flexibility (having resiliency). In the present invention, the diameter of the light radiating probe 40 may be, but not limited thereto, a diameter (φ0.3 mm) narrower than the diameter illustrated in Fig. 4(a), or may be substantially a value which falls between 0.3 mm and 5 mm. The core member 42 of the light radiating probe 40 may be made of, besides the above-mentioned acrylic resin, polyethylene, polypropylene, silicon, polyvinylchloride(PVC), Teflon, polyvinyl alcohol, polyvinyl butyral, polyimide, polyurethane, nylon, various polyesters, polyethylene naphthalate, polyethylene terephthalate or the like. However, a material for forming the core member 42 is not limited to these materials.

The light radiating probe 40 according to the present invention is formed of an optical fiber having high flexibility and hence, the invasiveness of the light radiating probe 40 when the light radiating probe 40 is inserted into a body cavity of a patient is low. Accordingly, a burden applied to a patient can be suppressed as much as possible. Shearing, breaking by bending or the like minimally occurs even when the light radiating probe 40 is used plural times and hence, the light radiating probe 40 can be used for a long period. Further, the light radiating probe 40 can be easily manufactured by applying roughening treatment or the like to the distal end portion as described previously.

Further, in the photodynamic therapy apparatus according to the present invention, in the case where a xenon light source or a tungsten light source having a broad spectrum distribution is used, unlike laser light source or a multicolor LED light source where an output wavelength region is limited, with the use of an arbitrary band pass filter, light which includes a wavelength region in which an arbitrary optimum excitation wavelength region of a photosensitizer (PS) is included at a peak can be selectively outputted. That is, light which corresponds to the photosensitizer (PS) can be outputted. Accordingly, the present invention is applicable to the PS molecular probe described in Patent Document 1 above.

### EXAMPLE 1

Using the photodynamic therapy apparatus 1 having the light radiating probe 40 according to the present invention, curing was applied to a colon/rectum cancer of a mouse in which a colon/rectum cancer was generated in the form of an autologous carcinogenesis (a cancer closest to a natural cancer) by photodynamic therapy (PDT).

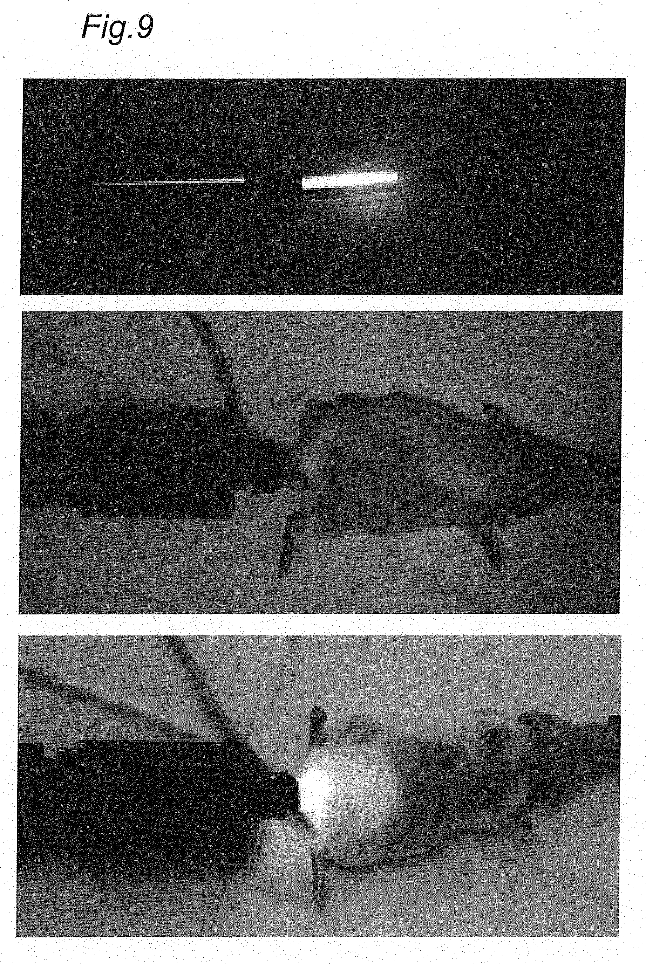

Fig. 9(a) shows the light radiating probe 40 according to the present invention extending from the joint jig 30. Fig. 9(a) shows a state where light from the xenon light source apparatus 10 propagated, and was radiated in all azimuth angles of 360° from the entire side surface of the light scattering and radiating portion 48 in a longitudinal direction. Fig. 9(b) shows a state where the light scattering and radiating portion 48 was inserted into the colon through an anus of a mouse into which a photosensitizer (PS) was injected by intravenous injection in advance. At this stage of the operation, the xenon light source apparatus 10 was not operated so that the light scattering and radiating portion 48 did not radiate light. Fig. 9(c) shows a state where the xenon light source apparatus 10 was operated from the state shown in Fig. 9(b) so that light was radiated to the colon/rectum cancer of the mouse from the light scattering and radiating portion 48. At this stage of the operation, it was confirmed that light was radiated to the entire abdomen of the mouse.

When photodynamic therapy (PDT) was continuously applied to the colon/rectum cancer for 10 min to 20 min once a week, it was confirmed three weeks later that the colon/rectum cancer of the mouse had substantially disappeared.

### DESCRIPTION OF REFERENCE SYMBOLS

10 LIGHT SOURCE APPARATUS
20 FLEXIBLE FIBER OPTICS (FIRST OPTICAL FIBER)
30 JOINT JIG
32 OPTICAL CONDENSER ADAPTER
34 LARGE-DIAMETER PORTION
35 NECK PORTION
36 SMALL-DIAMETER PORTION
37 INCIDENT END SURFACE
38 EMITTING END SURFACE
40 LIGHT RADIATING PROBE FOR PHOTODYNAMIC THERAPY (SECOND OPTICAL FIBER)
42 CORE MEMBER
44 CLADDING
46 LIGHT GUIDE PORTION
48 LIGHT SCATTERING AND RADIATING PORTION
60 ROD
62 SHEATH (COVER FILM)
100 CONVENTIONAL LIGHT RADIATING PROBE
102 ILLUMINATION LENS
104 OBJECT LENS (INCLUDING A CCD ELEMENT)
106 FORCEPS OPENING
108 NOZZLE
200 COLON
202 SUPERFICIAL CANCER TISSUE
204 LOWER LAYER CANCER TISSUE

## Claims

1. A light radiating probe for photodynamic therapy comprising an optical fiber which extends in an axial direction and through which light from a light source propagates, wherein
the optical fiber has
a light guide portion which is formed by forming thin film cladding on a side surface of a flexible core, and
a light scattering and radiating portion which is configured to scatter, with uniform intensity, light propagating through the light guide portion to a periphery of the light scattering and radiating portion in all azimuth angles with respect to an axial direction of the flexible core.

2. The light radiating probe for photodynamic therapy according to claim 1, wherein
the light scattering and radiating portion has a length which corresponds to a length of 1 cm or more of an affected part therapy target portion in an axial direction and is configured to radiate the light propagating from the light guide portion to an entire area near the affected part therapy target portion in all azimuth angles of 360 degrees, and
a peak wavelength of the light from the light source is included in an optimum excitation wavelength region of a desired photosensitizer used in photodynamic therapy.

3. The light radiating probe for photodynamic therapy according to claim 1 or 2, wherein the light scattering and radiating portion further includes a spirally wound rod.

4. The light radiating probe for photodynamic therapy according to claim 3, wherein the rod is a rod-shaped endoscopic fiberscope.

5. The light radiating probe for photodynamic therapy according to claim 3 or 4, further comprising a covering part which covers the light scattering and radiating portion and the rod.

6. A photodynamic therapy apparatus including a light source which radiates light, the photodynamic therapy apparatus comprising:
a first optical fiber through which light from the light source propagates and including an emitting end surface which has a first cross-sectional area;
an optical condenser adapter having
a condenser incident end surface through which light from the first optical fiber propagates and which is substantially adapted to the emitting end surface of the first optical fiber, and
a condenser emitting end surface which is smaller than the emitting end surface of the first optical fiber and is substantially adapted to an incident end surface of a second optical fiber; and
the second optical fiber through which the light from the optical condenser adapter propagates and including an incident end surface substantially adapted to a second cross-sectional area of the emitting end surface of the optical condenser adapter, wherein
the second optical fiber has
a light guide portion which is formed by forming a thin film cladding on a side surface of a flexible core, and
a light scattering and radiating portion configured to scatter, with uniform intensity, light which propagates through the light guide portion to a periphery of the light scattering and radiating portion in all azimuth angles with respect to an axial direction of a flexible core.

7. The photodynamic therapy apparatus according to claim 6, wherein
the light scattering and radiating portion has a length which corresponds to a length of 1 cm or more of an affected part therapy target portion in an axial direction is configured to radiate the light propagating from the light guide portion to an entire area near the affected part therapy target portion in all azimuth angles of 360 degrees, and
a peak wavelength of the light from the light source is included in an optimum excitation wavelength region of a desired photosensitizer used in photodynamic therapy.

8. The photodynamic therapy apparatus according to claim 6 or 7, wherein
the first and second optical fibers are formed of a plastic optical fiber having flexibility, and
the optical condenser adapter has
a glass core whose cross-sectional area is continuously decreased between an incident end surface having a first cross-sectional area and an emitting end surface having a second cross-sectional area, and
a thin film cladding which is formed on a side surface of the glass core.

9. The photodynamic therapy apparatus according to any one of claims 6 to 8, wherein the light scattering and radiating portion of the second optical fiber further includes a spirally wound rod.

10. The photodynamic therapy apparatus according to claim 9, wherein the rod is a rod-shaped endoscopic fiberscope.

11. The photodynamic therapy apparatus according to claim 9 or 10, further comprising a covering part which covers the light scattering and radiating portion and the rod.

12. A method of manufacturing a light radiating probe for photodynamic therapy, the method comprising the steps of:
providing an optical fiber which is formed by forming a thin film cladding on a side surface of a flexible core;
forming a light scattering and radiating portion by processing a side surface of a distal end portion of the optical fiber so as to scatter, with uniform intensity, light which propagates to the optical fiber at a distal end portion of the optical fiber in all azimuth angles; and
winding the light scattering and radiating portion around a rod.

13. The method according to claim 12, wherein
the light scattering and radiating portion has a length which corresponds to a length of 1 cm or more of an affected part therapy target portion in an axial direction is configured to radiate the light propagating from the light guide portion to an entire area near the affected part therapy target portion in all azimuth angles of 360 degrees, and
a peak wavelength of the light from the light source is included in an optimum excitation wavelength region of a desired photosensitizer used in photodynamic therapy.

14. The method according to claim 12, wherein the step of forming the light scattering and radiating portion by processing the side surface of the distal end portion of the optical fiber includes any one of the steps of:
exposing the flexible core by removing the thin film cladding disposed on the side surface of the distal end portion of the optical fiber and roughening a surface of the flexible core;
making the side surface of the thin film cladding disposed on the side surface of the distal end portion of the optical fiber into milky color using a solvent,; and
adhering fine powder on the side surface of the flexible core exposed by removing the thin film cladding disposed on the side surface of the distal end portion of the optical fiber.

15. The method according to any one of claims 12 to 14, further comprising a step of covering the light scattering and radiating portion and the rod with a resin material.
